# EUROPÄISCHE PATENTSCHRIFT

(11) **EP 2 445 510 B1**
(45) Veröffentlichungstag und Bekanntmachung des Hinweises auf die Patenterteilung: **27.12.2017**
(21) Anmeldenummer: 10725744.6
(22) Anmeldetag: 18.06.2010
(51) Int. Cl.: A61K 36/738, A61K 38/39, A61K 45/06, A61P 19/02, A23L 33/18, A23L 33/105

(54) **ZUSAMMENSETZUNG ZUR BEHANDLUNG VON DEGENERATIVEN GELENKERKRANKUNGEN**
COMPOSITIONS FOR TREATING DEGENERATIVE JOINT DISEASES
COMPOSITION POUR LE TRAITEMENT DE L'OSTÉOARTHRITE

(30) Priorität: 22.06.2009 DE 102009030351
(43) Veröffentlichungstag der Anmeldung: 02.05.2012
(73) Patentinhaber: GELITA AG, 69412 Eberbach (DE)
(72) Erfinder: OESSER, Steffen, 24960 Glücksburg (DE)
(74) Vertreter: Hoeger, Stellrecht & Partner Patentanwälte mbB
(86) Internationale Anmeldenummer: PCT/EP2010/058674
(87) Internationale Veröffentlichungsnummer: WO 2010/149596

(56) Entgegenhaltungen:
- WO-A1-2008/003314
- WO-A2-2008/006589
- WO-A2-2009/080778
- US-A1- 2006 198 810
- WINTHER ET AL: "P18 A powder made from subspecies of Rose-hip may act structure- modifying in osteoarthritis" OSTEOARTHRITIS AND CARTILAGE, BAILLIERE TINDALL, LONDON, GB LNKD- DOI:10.1016/S1063-4584(07)61373-9, Bd. 15, 1. Januar 2007 (2007-01-01), Seite B92, XP022221536 ISSN: 1063-4584
- WINTHER ET AL: "A standardized powder made from rosehips (Rosa canina L.) improves function and reduces pain and the consumption of rescue medication in osteoarthritis" OSTEOARTHRITIS AND CARTILAGE, BAILLIERE TINDALL, LONDON, GB LNKD- DOI:10.1016/S1063-4584(08)60004-7, Bd. 16, 1. April 2008 (2008-04-01), Seiten S8-S9, XP022663036 ISSN: 1063-4584 [gefunden am 2008-04-01]
- DEAL C L ET AL: "NUTRACEUTICALS AS THERAPEUTIC AGENTS IN OSTEOARTHRITIS THE ROLE OF GLUCOSAMINE, CHONDROITIN SULFATE, AND COLLAGEN HYDROLYSATE" RHEUMATIC DISEASES CLINICS OF NORTH AMERICA, W.B. SAUNDERS, PHILADELPHIA, PA, US LNKD- DOI:10.1016/S0889-857X(05)70074-0, Bd. 25, Nr. 2, 1. Mai 1999 (1999-05-01), Seiten 379-395, XP009050485 ISSN: 0889-857X
- BELLO A E ET AL: "Collagen hydrolysate for the treatment of osteoarthritis and other joint disorders: A review of the literature" CURRENT MEDICAL RESEARCH AND OPINION, INFORMA HEALTHCARE, GB LNKD- DOI:10.1185/030079906X148373, Bd. 22, Nr. 11, 1. November 2006 (2006-11-01), Seiten 2221-2232, XP009132739 ISSN: 0300-7995 [gefunden am 2006-10-10]
- "[Treatment of arthrosis with collagen hydrolysate. From empiricism to evidence--an effective therapeutic principle comes through]" MMW FORTSCHRITTE DER MEDIZIN 20 NOV 2003 LNKD- PUBMED:14725036, Bd. 145, Nr. 47, 20. November 2003 (2003-11-20), Seiten 58-59, XP001525337 ISSN: 1438-3276
- DATABASE BIOTECHABS/CA [Online] OSZMIANSKI J ET AL: "Possible use of enzymatic preparations in the production of cloudy juices of high vitamin content from fruits of the rose Rosa rugosa; rosehip pectin degradation and juice preparation with high ascorbic a cid content" XP002490528 gefunden im BIOTECHABS/CA PAN - 1993-11708
- WILLICH S N ET AL: "Rose hip herbal remedy in patients with rheumatoid arthritis a randomised controlled trial" PHYTOMEDICINE, GUSTAV FISCHER VERLAG, STUTTGART, DE, Bd. 17, Nr. 2, 1. Februar 2010 (2010-02-01), Seiten 87-93, XP026832079 ISSN: 0944-7113 [gefunden am 2009-10-08]

## Beschreibung

Die vorliegende Erfindung betrifft eine Zusammensetzung, insbesondere in Form eines Arzneimittels oder eines Nahrungsergänzungsmittels, zur Behandlung von degenerativen Gelenkerkrankungen.

Degenerative Gelenkerkrankungen, die durch Verschleiß oder Schädigung des Gelenkknorpels gekennzeichnet sind, werden meist unter dem Begriff Arthrose zusammengefasst. Teilweise wird auch der aus der englischsprachigen Literatur übernommene Begriff Ostheoartrithis synonym verwendet. Degenerative Gelenkerkrankungen sind in der gesamten Bevölkerung weit verbreitet, wobei die Häufigkeit des Auftretens mit dem Alter stark zunimmt. Die am häufigsten betroffenen Gelenke sind die Knie- und Hüftgelenke.

Die Ursachen des Gelenkverschleißes sind unterschiedlich und können sowohl in einer biologischen Minderwertigkeit des Knorpelgewebes liegen (primäre Arthrose) als auch in Einflussfaktoren wie mechanischer Überlastung (z.B. durch Gelenkfehlstellung), Entzündungen oder Stoffwechselstörungen (sekundäre Arthrose). In jedem Fall kann es bei fortschreitender Erkrankung zu einem vollständigen Verlust des Gelenkknorpels kommen, sodass die subchondralen Knochenflächen freiliegen und aneinander reiben, was bei den betroffenen Patienten zu erheblichen Beschwerden führt.

Knorpelgewebe besteht zum überwiegenden Teil aus einer extrazellulären Matrix, deren Bestandteile durch die in der Matrix verteilten Chondrocyten (Knorpelzellen) synthetisiert werden. Im Fall des Gelenkknorpels (Hyalinknorpel) umfasst die Trockenmasse der extrazellulären Matrix ca. 60 Gew.% Kollagen (überwiegend vom Typ II), ca. 25 bis 35 Gew% Proteoglykane sowie 15 bis 20 Gew.% nicht-kollagenöse Proteine. Der Auf- und Abbau der extrazellulären Matrix folgt einem komplexen Regulationsmechanismus, wobei in gesundem Knorpelgewebe die Chondrocyten auf eine geänderte Zusammensetzung der Matrix reagieren, indem die Biosynthese der Matrixkomponenten entsprechend reguliert wird. Die Versorgung des Knorpelgewebes mit Nährstoffen erfolgt dabei nur durch die periodischen Druckänderungen innerhalb des Gelenks, da im Knorpel weder Blut- noch Lymphgefäße vorhanden sind.

Es ist seit Längerem bekannt, degenerative Gelenkerkrankungen durch die orale Verabreichung von Kollagenhydrolysat zu behandeln. Zur Wirksamkeit dieser Behandlung im Hinblick auf eine Linderung der Beschwerden wurden mittlerweile eine Reihe von klinischen Untersuchungen durchgeführt (siehe A. Bello und S. Oesser (2006), Current Medical Research and Opinion (22) 2221-2232). Obwohl die exakte Wirkungsweise des Kollagenhydrolysats noch nicht geklärt ist, geht man davon aus, dass die Kollagenfragmente in den Blutkreislauf resorbiert werden und im Knorpelgewebe die Synthese der extrazellulären Matrix stimulieren, indem sie in den oben beschriebenen Regulationsmechanismus eingreifen. Durch *in vitro-*Versuche mit Chondrocyten konnte nachgewiesen werden, dass die Biosynthese und Sekretion von Kollagen in Anwesenheit von Kollagenhydrolysat erhöht ist (siehe S. Oesser und J. Seifert (2003), Cell Tissue Research (311) 393-399).

Die nachveröffentlichte internationale Patentanmeldung WO 2009/080778 A2 offenbart eine Zusammensetzung enthaltend einen entzündungshemmenden Pflanzenextrakt aus Hagebutte zusammen mit einer knorpelprotektiven Substanz, die u.a. aus Kollagenhydrolysaten ausgewählt ist, sowie ein Verfahren zur Herstellung des Hagebuttenextraktes.

Der Erfindung liegt die Aufgabe zugrunde, eine Zusammensetzung zur Behandlung von degenerativen Gelenkerkrankungen mit einer verbesserten Wirksamkeit zur Verfügung zu stellen.

Diese Aufgabe wird erfindungsgemäß gelöst durch eine Zusammensetzung, die Kollagenhydrolysat und Hagebuttenpulver und/oder -extrakt umfasst, wobei das Gewichtsverhältnis von Kollagenhydrolysat zu Hagebuttenpulver und/oder -extrakt, jeweils bezogen auf die Trockenmasse, im Bereich von ca. 10:1 bis ca. 20:1 liegt.

Es wurde überraschenderweise gefunden, dass die stimulierende Wirkung von Kollagenhydrolysat auf die Biosynthese von Kollagen und Proteoglykanen in den Chondrocyten durch eine Kombination mit Hagebuttenpulver oder -extrakt verstärkt werden kann. Dieser Wirkung liegt ein synergetischer Effekt zugrunde, da eine solche Stimulierung durch Hagebuttenpulver oder -extrakt allein nicht beobachtet wird.

Die medizinische Anwendung von Hagebutten, auch im Zusammenhang mit Arthrose, ist an sich bekannt. Diese Anwendung beruht jedoch auf einer entzündungshemmenden Wirkung eines insbesondere in Hagebuttenpulver enthaltenen Galaktolipids (GOPO). Entzündungen können Ursache und/oder Begleiterscheinung von degenerativen Gelenkerkrankungen sein, und es gibt Studien, die eine Schmerzlinderung durch die Einnahme von Hagebuttenpulver zeigen (siehe D. Warholm et al. (2003), Current Therapeutic Research (64) 21-31). Ein Einfluss von Hagebuttenpulver oder -extrakt auf die Biosynthese von Knorpelbestandteilen wurde bisher jedoch nicht beschrieben.

Bei der erfindungsgemäßen Zusammensetzung liegt das Gewichtsverhältnis von Kollagenhydrolysat zu Hagebuttenpulver und/oder -extrakt, jeweils bezogen auf die Trockenmasse, im Bereich von ca.10:1 bis ca. 20:1. Bei Gewichtsverhältnissen oberhalb von ca. 100:1, d.h. bei Verwendung von weniger als ca. 1 Gew.% Hagebuttenpulver und/oder -extrakt, bezogen auf das Kollagenhydrolysat, ist der oben beschriebene synergetische Effekt in der Regel nicht nachweisbar. Andererseits kann durch die Verwendung von mehr als ca.

50 Gew.% Hagebuttenpulver und/oder -extrakt, bezogen auf das Kollagenhydrolysat (d.h. bei einem Verhältnis unterhalb von ca. 2:1), nur noch eine unwesentliche oder überhaupt keine Steigerung dieses Effekts mehr erreicht werden. Der beschriebene Effekt kann also mit einem Unterschuss an Hagebuttenpulver und/oder -extrakt erreicht werden.

Unter Kollagenhydrolysat wird im Rahmen der vorliegenden Erfindung jedes Hydrolyseprodukt von Kollagen verstanden, das aufgrund eines ausreichend geringen Molekulargewichts unter den Bedingungen des Standard-Bloomtests nicht mehr geliert, d.h. eine Gelstärke von 0 g Bloom aufweist. Vorzugsweise ist das Kollagenhydrolysat durch enzymatische Hydrolyse von Kollagen des Typs I und/oder des Typs II gewonnen. Es hat sich gezeigt, dass der stimulierende Effekt auf die Biosynthese der extrazellulären Matrix des Knorpelgewebes (welches überwiegend aus Kollagen des Typs II besteht) im Wesentlichen unabhängig ist vom Typ des Kollagenhydrolysats, was darauf hindeutet, dass die Peptidfragmente in Kollagenhydrolysat des Typs I und des Typs II die gleiche oder eine ähnliche Struktur aufweisen.

Als Ausgangsmaterial für die Herstellung von Kollagenhydrolysat eignen sich insbesondere Knochen, Haut, Bindegewebe (jeweils Typ I) oder Knorpel (Typ II) verschiedener Tiere. Typische Ausgangsmaterialien sind z.B. Rinderknochen, Rinderspalt und Schweineschwarte, es kann aber auch Kollagenhydrolysat z.B. aus Kollagen von Geflügel oder Fischen gewonnen werden. Das Herstellungsverfahren für das Kollagenhydrolysat kann dabei sowohl von nativem Kollagen ausgehen als auch von Gelatine, also extrahiertem und denaturiertem Kollagen.

Das Kollagenhydrolysat in der erfindungsgemäßen Zusammensetzung weist bevorzugt ein mittleres Molekulargewicht von ca. 0,3 kDa bis ca. 30 kDa auf, insbesondere von ca. 2 kDa bis ca. 10 kDa. Die Molekulargewichtsverteilung des Kollagenhydrolysats kann durch die Auswahl geeigneter proteolytischer Enzyme (meist bakterielle Proteasen) gesteuert werden. Zusätzlich können gegebenenfalls einzelne Molekulargewichtsfraktionen des Hydrolysats durch Ultrafiltration oder chromatographische Verfahren abgetrennt werden.

Bei dem Hagebuttenpulver und/oder -extrakt in der erfindungsgemäßen Zusammensetzung kann es sich im weitesten Sinn um alle Produkte handeln, die aus Hagebutten gewonnen werden. Hagebutten sind die Scheinfrüchte verschiedener Rosenarten, wobei bevorzugt die Hagebutten der Hundsrose (*Rosa canina)* verwendet werden. Sie bestehen im Wesentlichen aus dem Fruchtfleisch bzw. der Schale (*Fructus cynosbati)* und den Samen (*Semen cynosbati),* bei denen es sich im botanischen Sinn um Nüsse handelt. Das Fruchtfleisch ohne die Nüsse wird auch als *Cynosbati sine semine* bezeichnet.

Gemäß einer Ausführungsform der Erfindung umfasst die Zusammensetzung ein Hagebuttenpulver aus *Fructus cynosbati* und/oder *Semen Cynosbati.* Unter einem Pulver ist dabei im Rahmen der Erfindung jedes im Wesentlichen homogene Produkt zu verstehen, das sämtliche Bestandteile der Hagebutten oder der verwendeten Teile der Hagebutten enthält, unabhängig von der Partikelgröße. Ein möglichst feinteiliges Pulver ist jedoch bevorzugt, da hiermit leichter eine homogene Mischung mit dem Kollagenhydrolysat und gegebenenfalls weiteren Komponenten der erfindungsgemäßen Zusammensetzung erhalten werden kann und die wirksamen Bestandteile der Hagebutte schneller in Lösung gehen können. Ein Hagebuttenpulver kann insbesondere durch Trocknen und Mahlen von *Fructus cynosbati* gewonnen werden, wie dies beispielsweise in der EP 1 071 439 B1 beschrieben ist.

Gemäß einer weiteren Ausführungsform der Erfindung umfasst die Zusammensetzung einen Hagebuttenextrakt aus *Fructus cynosbati* und/oder *Semen cynosbati.* Unter einem Extrakt ist dabei im Rahmen der Erfindung jegliches Produkt zu verstehen, das durch Extraktion der Hagebutten oder der verwendeten Teile der Hagebutten mit einem fluiden Extraktionsmittel gewonnen wird. Das oben definierte Gewichtsverhältnis des Hagebuttenextrakts zu dem Kollagenhydrolysat bezieht sich dabei auf die Trockenmasse der extrahierten Bestandteile ohne das Extraktionsmittel. Die Verwendung eines Extrakts ist gegenüber der Verwendung eines Pulvers bevorzugt, da die Zusammensetzung eines Extrakts in der Regel besser reproduzierbar ist. Des Weiteren kann ein Hagebuttenextrakt auch unmittelbar in flüssiger Form als Bestandteil einer flüssigen erfindungsgemäßen Zusammensetzung verwendet werden, was bei einem Hagebuttenpulver nicht möglich ist. Wird ein getrockneter Extrakt verwendet, so kann dieser besser dispergiert bzw. gelöst werden als ein Hagebuttenpulver.

Besonders günstig ist es, wenn der Hagebuttenextrakt durch Extraktion von *Fructus cynosbati* mit einem wässrigen Extraktionsmittel, welches bis zu 50 Vol.% Ethanol enthält, gewonnen ist. Es kann insbesondere auch ein rein wässriges Extraktionsmittel ohne Ethanol eingesetzt werden, sodass der gewonnene Hagebuttenextrakt vollständig und gut wasserlöslich ist. Interessanterweise hat sich gezeigt, dass derartige wässrige oder wässrig/alkoholische Hagebuttenextrakte, die im Rahmen der vorliegenden Erfindung äußerst wirksam sind, häufig keine nachweisbaren Mengen des Galaktolipids GOPO enthalten, welches für die entzündungshemmende Wirkung bekannter Hagebuttenpräparate verantwortlich gemacht wird. Dies deutet darauf hin, dass der gefundene synergetische Effekt, d.h. die Steigerung der Wirkung des Kollagenhydrolysats auf die Biosynthese von Matrixkomponenten, auf andere, bisher nicht identifizierte Bestandteile bzw. Inhaltsstoffe der Hagebutte zurückzuführen ist.

Der Hagebuttenextrakt kann vor der Verwendung in der erfindungsgemäßen Zusammensetzung verschiedenen Nachbehandlungen unterzogen werden. Eine solche Nachbehandlung umfasst bevorzugt eine Enzymbehandlung und/oder eine Membranfiltration. Durch diese Verfahren können unerwünschte Bestandteile des Extraktes abgebaut bzw. entfernt werden, wodurch die Wirksamkeit des Extrakts unter Umständen erhöht werden kann.

Die Enzymbehandlung umfasst vorzugsweise eine Behandlung des Hagebuttenextrakts mit einem oder mehreren Enzymen, die ausgewählt sind aus Glykosidasen, Cellulasen und Pektinasen. Solche Enzymmischungen werden unter anderem auch zur Klärung von Wein eingesetzt und sind z.B. unter dem Markennamen Ultrazym® erhältlich.

Die Membranfiltration umfasst bevorzugt eine Filtration des Hagebuttenextrakts bei einer Ausschlussgröße von ca. 1 kDa bis ca. 500 kDa, insbesondere von ca. 10 kDa bis ca. 300 kDa. Hierbei werden Stoffe mit einem Molekulargewicht oberhalb der jeweiligen Ausschlussgröße abgetrennt und das Permeat für die erfindungsgemäße Zusammensetzung weiterverwendet.

Gegenstand der vorliegenden Erfindung ist ferner ein Arzneimittel oder Nahrungsergänzungsmittel, welches die vorstehend beschriebene Zusammensetzung, umfassend Kollagenhydrolysat und Hagebuttenpulver oder -extrakt, umfasst. Insbesondere betrifft die Erfindung ein solches Arzneimittel oder Nahrungsergänzungsmittel zur Behandlung oder Vorbeugung von degenerativen Gelenkerkrankungen.

Da die wesentlichen Bestandteile der erfindungsgemäßen Zusammensetzung tierischen bzw. pflanzlichen Ursprungs sind, ist eine Zulassung als Arzneimittel nicht erforderlich und die Zusammensetzung kann daher auch als Nahrungsergänzungsmittel deklariert und angeboten werden, wie dies im Fall von Kollagenhydrolysat seit längerem üblich ist. Von schädlichen Nebenwirkungen der erfindungsgemäßen Zusammensetzung ist nach heutigem Kenntnisstand nicht auszugehen.

Die degenerativen Gelenkerkrankungen, die mit dem erfindungsgemäßen Arzneimittel oder Nahrungsergänzungsmittel behandelt werden können, umfassen insbesondere Arthrose in allen Erscheinungsformen, und allgemein alle Erkrankungen, die mit einer Schädigung bzw. einem Verschleiß von Knorpelgewebe einhergehen, insbesondere rheumatoide Arthritis, Erkrankungen des rheumatischen Formenkreises, Spondylitis und Fibromyalgie.

Aufgrund seiner Wirkungsweise eignet sich das erfindungsgemäße Arzneimittel oder Nahrungsergänzungsmittel nicht nur zur Behandlung von akuten Erkrankungen, sondern auch zu deren Vorbeugung, insbesondere bei Personen, die aufgrund einer Stoffwechselstörung oder einer überdurchschnittlichen Belastung der Gelenke (z.B. Sportler) besonders gefährdet sind. Unter diesem Aspekt betrifft die Erfindung auch ein Arzneimittel oder Nahrungsergänzungsmittel der oben beschriebenen Art zur Stimulierung der Bildung von Knorpelgewebe.

Das Arzneimittel oder Nahrungsergänzungsmittel kann in Form einer Lösung, eines Sirups, eines Pulvers oder eines Granulats vorliegen. Bei Verwendung eines Hagebuttenpulvers in der erfindungsgemäßen Zusammensetzung liegt diese insgesamt in fester Form vor, während bei Verwendung eines Hagebuttenextrakts sowohl eine flüssige als auch eine feste Zusammensetzung als Arzneimittel oder Nahrungsergänzungsmittel formuliert werden kann. Die genannten Darreichungsformen ermöglichen eine individuelle Dosierung durch den Anwender, alternativ kann z.B. auch eine Lösung vordosiert in Ampullen angeboten werden.

Alternativ kann das Arzneimittel oder Nahrungsergänzungsmittel auch in fester, vordosierter Form vorliegen, also insbesondere in Form von Tabletten, Filmtabletten, Kapseln, Pastillen oder Dragees. Günstigerweise umfasst eine Dosis des Arzneimittels oder des Nahrungsergänzungsmittels ca. 0,1 g bis ca. 20 g Kollagenhydrolysat, insbesondere ca. 1 g bis 10 g, was einer bevorzugten Tagesdosis entspricht. Beispielsweise kann eine Dosis 10 g Kollagenhydrolysat umfassen und dementsprechend von ca. 0,1 g bis ca. 5 g Hagebuttenpulver und/oder -extrakt.

Diese und weitere Vorteile der Erfindung werden anhand der folgenden Beispiele unter Bezugnahme auf die Figuren näher erläutert. Es zeigen im Einzelnen:
- Figur 1:: Diagramm zur Biosynthese von Kollagen (Hagebuttenextrakt);
- Figur 2:: Diagramm zur Biosynthese von Proteoglykanen (Hagebuttenextrakt);
- Figur 3:: Diagramm zur Expression von Kollagen-RNA (Hagebuttenextrakt);
- Figur 4:: Diagramm zur Expression von Aggrecan-RNA (Hagebuttenextrakt);
- Figur 5:: Diagramm zur Biosynthese von Kollagen (Hagebuttenextrakt);
- Figur 6:: Diagramm zur Biosynthese von Proteoglykanen (Hagebuttenextrakt);
- Figur 7:: Diagramm zur Biosynthese von Kollagen (Hagebuttenpulver);
- Figur 8:: Diagramm zur Biosynthese von Proteoglykanen (Hagebuttenpulver);
- Figur 9:: Diagramm zur Expression von Kollagen-RNA (Hagebuttenpulver);
- Figur 10:: Diagramm zur Expression von Aggrecan-RNA (Hagebuttenpulver);
- Figur 11:: Diagramm zur Biosynthese von Kollagen (Hagebuttenpulver); und
- Figur 12:: Diagramm zur Biosynthese von Proteoglykanen (Hagebuttenpulver).

Die synergetische Wirkung der erfindungsgemäßen Zusammensetzung auf die Biosynthese der wesentlichen Matrixkomponenten des Knorpelgewebes wurde anhand eines *in vitro*-Modells mit porcinen und humanen Chondrozyten nachgewiesen.

### Kollagenhydrolysat

Das für die Versuche verwendete Kollagenhydrolysat weist ein mittleres Molekulargewicht von ca. 3 kDa auf und wird durch Hydrolyse von tierischem Kollegen des Typs I mit Hilfe von bakteriellen Proteasen hergestellt. Dieses Kollagenhydrolysat wird von der Anmelderin unter dem Namen Fortigel® als Nahrungsergänzungsmittel zur Stimulierung der Bildung von Knorpelgewebe vertrieben.

### Hagebuttenextrakt

Es wurde ein wässriger Extrakt aus Hagebuttenschalen (*Fructus cynosbati*) der Hundsrose verwendet. Ein solcher Hagebuttenextrakt kann hergestellt werden, indem z.B. 1 kg Hagebuttenschalen zweimal mit je 6 I Wasser bei 50 °C für eine Dauer von 6 h extrahiert werden. Nach dem Absetzenlassen werden die Extraktlösungen vereinigt und klarfiltriert. Nach dem Einrotieren des Extraktionsmittels kann der Extrakt bei 50 °C im Vakuum getrocknet werden.

Die Trockenmasse des auf diese Weise erhaltenen Hagebuttenextrakts entspricht einer Ausbeute im Bereich von ca. 35 bis 45%. Der Extrakt enthält keine nachweisbaren Mengen des Galaktolipids GOPO.

### Hagebuttenpulver

Es wurde ein nach einem standardisierten Verfahren hergestelltes Hagebuttenpulver verwendet, das unter dem Namen LITOZIN® von der Queisser Pharma GmbH & Co. KG (Flensburg) vertrieben wird. Dieses Hagebuttenpulver enthält relativ große Mengen des Galaktolipids GOPO.

### Kultivierung von Chondrocyten in vitro

Für die Zellkulturen wurden porcine bzw. humane Chondrocyten auf bekannte Weise aus Knorpelgewebe isoliert und mit einer Dichte von ca. 350.000 Zellen/cm² auf Kulturplatten ausgesät. Als Kulturmedium wurde Ham's F12-Medium mit 10% fötalem Kälberserum, 10 µg/ml Gentamycin und 5 µg/ml Amphotericin B verwendet. Die Kultivierung erfolgte bei 37 °C in einer sauerstoffreduzierten Atmosphäre (5% O₂, 5% CO₂ und 90% N₂).

Je nach Ansatz (siehe unten) wurden dem Kulturmedium Kollagenhydrolysat und/oder Hagebuttenextrakt zugesetzt.

### Bestimmung der Kollagen-Biosynthese

Die Quantifizierung des von den Chondrocyten synthetisierten Kollagens (im Wesentlichen Typ II) erfolgt durch radioaktive Markierung mit ¹⁴C-Prolin, welches in das Kollagen eingebaut wird.

Dem Kulturmedium wird zunächst radioaktives ¹⁴C-Prolin zugesetzt und die Chondrocyten werden unter diesen Bedingungen bis zum Zeitpunkt der Bestimmung kultiviert. Um bei der Detektion das eingebaute von nicht eingebautem ¹⁴C-Prolin unterscheiden zu können, wird das isotopenhaltige Kulturmedium dann für einen Zeitraum von 3 Tagen durch reines Kulturmedium ersetzt. Anschließend wird das Kulturmedium verworfen und der adhärente Zellrasen wird mit destilliertem Wasser versetzt, um durch osmotischen Stress die Zellmembranen zu zerstören und cytosolisches, nicht gebundenes ¹⁴C-Prolin freizusetzen. Durch Zentrifugation werden die Zelltrümmer mit der synthetisierten extrazellulären Matrix pelletiert. Das Pellet wird in frischem destillierten Wasser resuspendiert und mit einem Xylen-Scintillations-Cocktail versetzt. Durch Detektion des ¹⁴C-Prolins mit einem Betacounter kann dann die Menge des synthetisierten Kollagens quantifiziert werden.

### Bestimmung der Proteoglykan-Biosynthese

Die Quantifizierung der von den Chondrocyten synthetisierten Proteoglykane erfolgt durch eine Alcianblau-Färbung und photometrische Bestimmung der Glykosaminoglykane (GAG), welche Bestandteile der Proteoglykane sind.

Um den Gehalt an GAG in der Zellkultur zu bestimmen, wird zunächst das Kulturmedium verworfen und der adhärente Zellrasen wird mit PBS-Puffer (pH 7) gespült. Anschließend werden die Zellen bei 4 °C für 2 Stunden in einer 10%igen Formaldehydlösung in PBS fixiert. Nach Entfernen des Formaldehyds wird das Alcianblau-Färbereagenz (5% Alcianblau in 3%iger Essigsäure) auf den Zellrasen gegeben und über Nacht bei 4 °C inkubiert. Nicht gebundenes Alcianblau wird verworfen und durch drei- bis viermaliges vorsichtiges Spülen mit PBS ausgewaschen. Durch Zugabe von saurer Guanidinlösung (8 mol/l) werden die GAG-Komplexe aus dem Zellrasen herausgelöst. Die Menge an Glykosaminoglykanen kann dann photometrisch bei einer Wellenlänge von 620 nm quantifiziert werden.

### Bestimmung der RNA-Expression von Kollagen und Aggrecan

Als mittelbarer Indikator für die Biosynthese von Kollagen und Proteoglykanen dient auch die Menge der exprimierten RNA, die für Kollagen vom Typ II bzw. für Aggrecan, einem wichtigen Proteoglykan, kodiert. Die Bestimmung erfolgt semiquantitativ in Relation zur RNA von Glycerinaldehyd-3-phosphat-Dehydrogenase (GAPDH).

Zunächst wird das Kulturmedium entfernt und durch Zugabe von Trizol® der Zellrasen aufgelöst. Die RNA wird in bekannter Weise mit Chloroform und Isopropanol isoliert. Die Menge an isolierter RNA wird bei 260 nm photometrisch bestimmt und 1 µg RNA werden anschließend durch reverse Transkription in cDNA umgeschrieben. Die spezifischen Sequenzen werden jeweils mit folgenden Primerpaaren (für porcine Chondrozyten) amplifiziert:
Kollagen Typ II:

| | |
|---|---|
| 5'-CCACTGCTCTCTGCCATACA-3' | (SEQ ID No. 1) |
| 5'-GTCCAGGTAGGCAATGCTGT-3' | (SEQ ID No. 2) |

Aggrecan:

| | |
|---|---|
| 5'-GGAGAAGAGATGCCAACAGC-3' | (SEQ ID No. 3) |
| 5'-ATGCTGCTCAGGTGTGACTG-3' | (SEQ ID No. 4) |

GAPDH:

| | |
|---|---|
| 5'-GGGCATGAACCATGAGAAGT-3' | (SEQ ID No. 5) |
| 5'-AAGCAGGGATGATGTTCTGG-3' | (SEQ ID No. 6) |

Die Amplifikation wird je Probe mit 1 µl cDNA, 19,65 µl dest. Wasser, 3 µl 10fach Gold-Puffer, 2,4 µl 25 mM MgCl₂, 0,6 mM dNTP, je 0,6 µl der spezifischen Primer und 0,15 µl AmpliTaq®-Polymerase durchgeführt. Das Thermocycler-Programm umfasst eine Initialisierung bei 95 °C für 10 min, eine Denaturierung bei 94 °C für 1 min, die Anlagerung der Primer bei 69 °C (Aggrecan), 64 °C (Typ II Kollagen) bzw. 58 °C (GAPDH) für 30 s und eine Elongation bei 72 °C für 30 s. Es werden jeweils 35 Zyklen durchgeführt, bevor die Polymerase bei 70 °C für 10 min inaktiviert wird. Die cDNA-Produkte werden dann auf einem 5%igen Agarose-Ethidiumbromid-Gel elektrophoretisch aufgetrennt und ihre Menge densitometrisch mit einem bildgebenden Verfahren ermittelt. Die Expression der Kollagen- und Aggrecan-RNA wird in Relation zur GAPDH-RNA semiquantitativ ausgewertet.

### Ergebnisse mit Hagebuttenextrakt

Die Biosynthese von Kollagen und von Proteoglykanen sowie die Expression von Kollagen-RNA und Aggrecan-RNA wurden für folgende Ansätze von Zellkulturen bestimmt und jeweils miteinander verglichen:

| | |
|---|---|
| Ansatz A: | Kontrolle (Kulturmedium ohne Kollagenhydrolysat oder Hagebuttenextrakt) |
| Ansatz B: | Kulturmedium mit 0,5 mg/ml Kollagenhydrolysat |
| Ansatz C: | Kulturmedium mit 0,035 mg/ml Hagebuttenextrakt (Trockensubstanz) |
| Ansatz D: | Kulturmedium mit 0,5 mg/ml Kollagenhydrolysat und 0,035 mg/ml Hagebuttenextrakt (Trockensubstanz) |

Für die Bestimmung der Biosynthese von Kollagen und Proteoglykanen wurden die Zellen jeweils 3 Tage unter den entsprechenden Bedingungen kultiviert, für die Bestimmung der RNA-Expression jeweils 1 Tag. Es wurden jeweils vergleichbare Resultate mit porcinen bzw. humanen Chondrozyten erhalten.

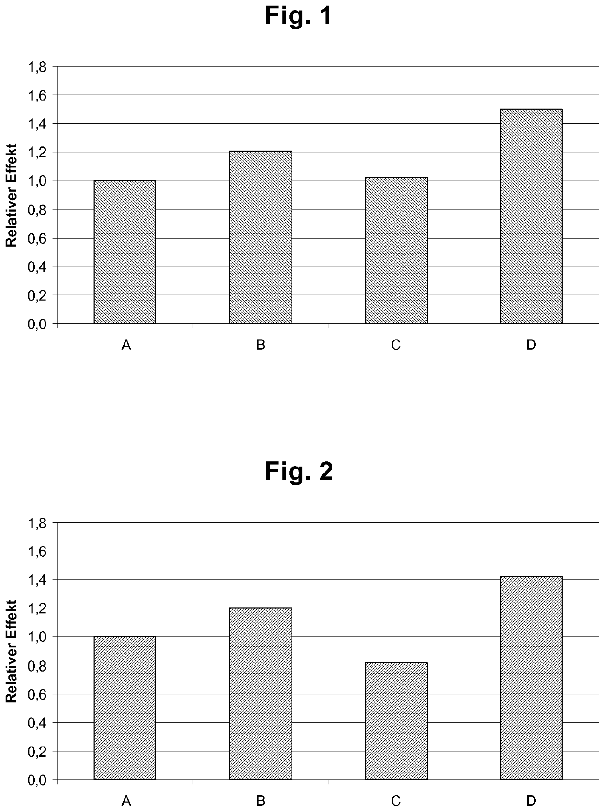

In der Figur 1 sind die Ergebnisse für die Biosynthese von Kollagen graphisch dargestellt, und zwar für die Ansätze B, C und D jeweils in Relation zum Ansatz A (Kontrolle = 1). Die Ergebnisse stellen die Mittelwerte aus jeweils mindestens sieben unabhängigen Bestimmungen dar.

Während die Biosynthese von Kollagen durch die Zugabe von Kollagenhydrolysat um ca. 20% gesteigert wird, führt die Zugabe von Hagebuttenextrakt allein zu keinem relevanten Effekt. Bei gleichzeitiger Zugabe von Kollagenhydrolysat und Hagebuttenextrakt (in einem Gewichtsverhältnis von ca. 14:1) wird jedoch eine Steigerung der Kollagenbiosynthese um ca. 50% gegenüber der Kontrolle beobachtet. Dieses Ergebnis macht deutlich, dass der Hagebuttenextrakt in Form eines synergetischen Effekts die stimulierende Wirkung des Kollagenhydrolysats auf die Kollagenbiosynthese verstärkt.

In der Figur 2 sind die entsprechenden Werte für die Biosynthese von Proteoglykanen dargestellt, wobei es sich um die Mittelwerte aus jeweils mindestens sechs unabhängigen Bestimmungen handelt. Während hier beim Ansatz C (Hagebuttenextrakt allein) sogar eine etwas geringere Proteoglykanbiosynthese als bei der Kontrolle beobachtet wird, sind die Steigerungsraten bei den Ansätzen B (Kollagenhydrolysat) und D (Kollagenhydrolysat mit Hagebuttenextrakt) vergleichbar mit denjenigen der Kollagenbiosynthese.

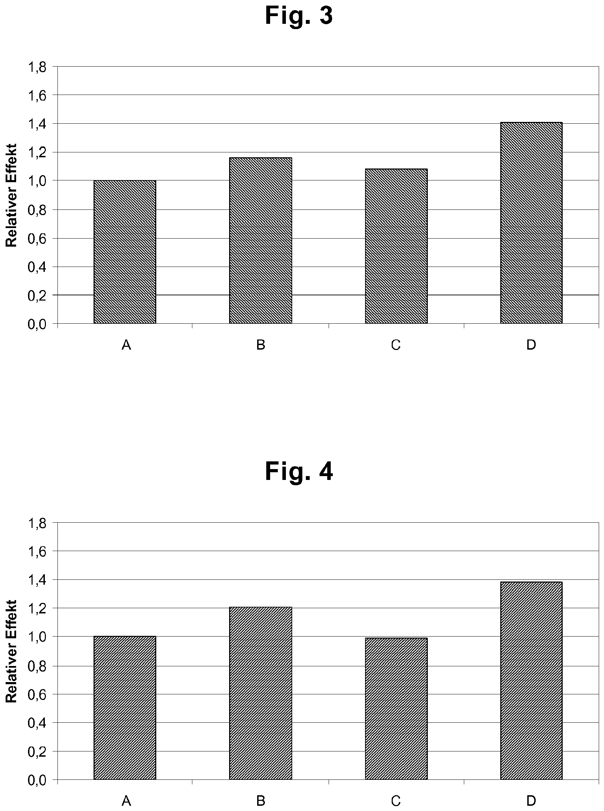

Auch auf der Ebene der Expression von RNA konnte diese Wirkung bestätigt werden. Die Figur 3 zeigt die Ergebnisse für die Expression von Kollagen-RNA (Mittelwerte aus jeweils 16 Bestimmungen) und die Figur 4 zeigt die Ergebnisse für die Expression von Aggrecan-RNA (Mittelwerte aus jeweils zehn Bestimmungen). In beiden Fällen stieg die RNA-Menge durch die Zugabe von Kollagenhydrolysat um ca. 20 % gegenüber der Kontrolle, und durch die Zugabe von Kollagenhydrolysat und Hagebuttenextrakt im Gewichtsverhältnis von ca. 14:1 um ca. 40 %.

Weitere Versuche wurden durchgeführt, um den Einfluss der Menge des eingesetzten Hagebuttenextraktes zu bestimmen. Hierzu wurden folgende Ansätze von Zellkulturen miteinander verglichen:

| | |
|---|---|
| Ansatz E: | Kontrolle (ohne Kollagenhydrolysat oder Hagebuttenextrakt) |
| Ansatz F: | 0,5 mg/ml Kollagenhydrolysat |
| Ansatz G: | 0,5 mg/ml Kollagenhydrolysat und 0,0035 mg/ml Hagebuttenextrakt |
| Ansatz H: | 0,5 mg/ml Kollagenhydrolysat und 0,035 mg/ml Hagebuttenextrakt |
| Ansatz I: | 0,5 mg/ml Kollagenhydrolysat und 0,35 mg/ml Hagebuttenextrakt |

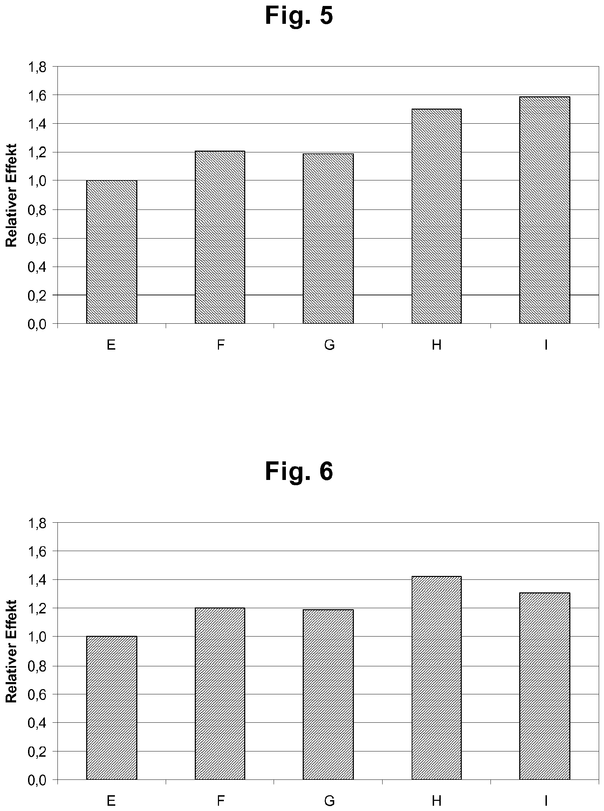

Die Figur 5 zeigt die Ergebnisse in Bezug auf die Biosynthese von Kollagen und die Figur 6 in Bezug auf die Biosynthese von Proteoglykanen (Mittelwerte aus jeweils mindestens sechs unabhängigen Bestimmungen). In beiden Fällen zeigt sich bei dem Ansatz G (Gewichtsverhältnis von Kollagenhydrolysat zu Hagebuttenextrakt von ca. 140:1) lediglich derselbe stimulierende Effekt von ca. 20% wie bei dem Ansatz F (nur Kollagenhydrolysat). Der Ansatz H (Gewichtsverhältnis ca. 14:1) zeigt die dem obigen Ansatz D entsprechenden Steigerungen von ca. 50% bzw. ca. 40%. Wird die Menge an Hagebuttenextrakt nochmals verzehnfacht (Ansatz I, Gewichtsverhältnis ca. 1,4:1), so ergibt sich bei der Kollagenbiosynthese (Figur 5) nur noch eine unterproportionale Steigerung auf knapp 60% gegenüber der Kontrolle, bei der Proteoglykanbiosynthese ist das Ergebnis sogar schlechter als bei dem Ansatz H.

### Ergebnisse mit Hagebuttenpulver

Die Biosynthese von Kollagen und von Proteoglykanen sowie die Expression von Kollagen-RNA und Aggrecan-RNA wurden für folgende Ansätze von Zellkulturen bestimmt und jeweils miteinander verglichen:

| | |
|---|---|
| Ansatz K: | Kontrolle (Kulturmedium ohne Kollagenhydrolysat oder Hagebuttenpulver |
| Ansatz L: | Kulturmedium mit 0,5 mg/ml Kollagenhydrolysat |
| Ansatz M: | Kulturmedium mit 0,05 mg/ml Hagebuttenpulver |
| Ansatz N: | Kulturmedium mit 0,5 mg/ml Kollagenhydrolysat und 0,5 mg/ml Hagebuttenpulver |

Die Kultivierung der Zellen und die anschließende Bestimmung der Biosynthese von Kollagen und Proteoglykanen sowie der RNA-Expression erfolgten jeweils wie bei der Verwendung von Hagebuttenextrakt (siehe oben).

Auch die im Folgenden beschriebenen Ergebnisse für porine Chondrozyten sind im Wesentlichen mit denjenigen vergleichbar, die bei Verwendung von Hagebuttenextrakt beobachtet wurden. Mit humanen Chondrocyten wurden ähnliche Ergebnisse erzielt.

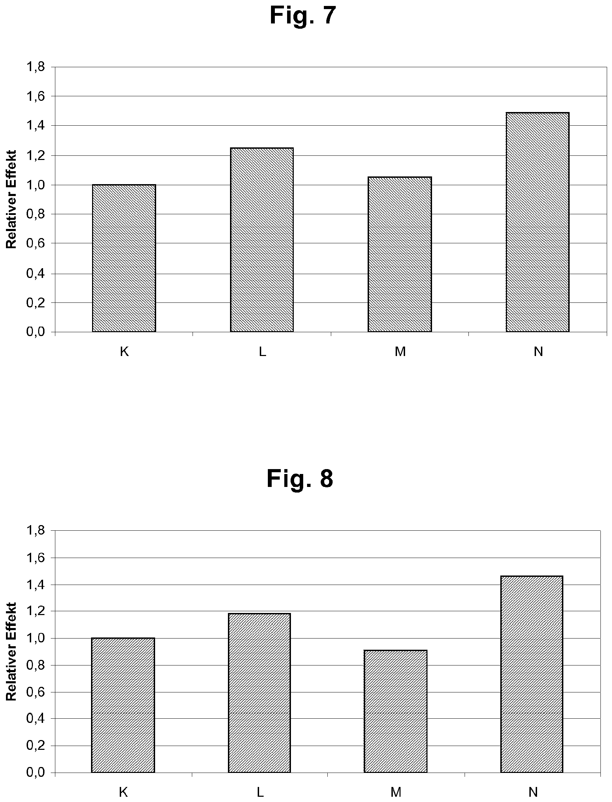

In der Figur 7 sind die Ergebnisse für die Biosynthese von Kollagen dargestellt, und zwar für die Ansätze L, M und N jeweils in Relation zum Ansatz K (Kontrolle = 1). Die Ergebnisse stellen die Mittelwerte aus jeweils mindestens sieben unabhängigen Bestimmungen dar. Die Biosynthese von Kollagen wird durch die Zugabe von Kollagenhydrolysat um ca. 25% gesteigert, durch die Zugabe von Hagebuttenpulver lediglich um ca. 5%. Bei gleichzeitiger Zugabe von Kollagenhydrolysat und Hagebuttenpulver (in einem Gewichtsverhältnis von 10:1) wird jedoch eine Steigerung um ca. 50% beobachtet.

Bei den in der Figur 8 dargestellten Werten für die Biosynthese von Proteoglykanen zeigt sich das Vorliegen eines synergetischen Effektes noch deutlicher. Der Zusatz von Hagebuttenpulver allein (Ansatz M) führt zu einer etwas geringeren Proteoglykan-Biosynthese als bei der Kontrolle (Ansatz K), während durch die Kombination von Hagebuttenpulver mit Kollagenhydrolysat (Ansatz N) eine etwa zweieinhalb Mal so hohe Steigerung als durch Kollagenhydrolysat allein (Ansatz L) erreicht wird.

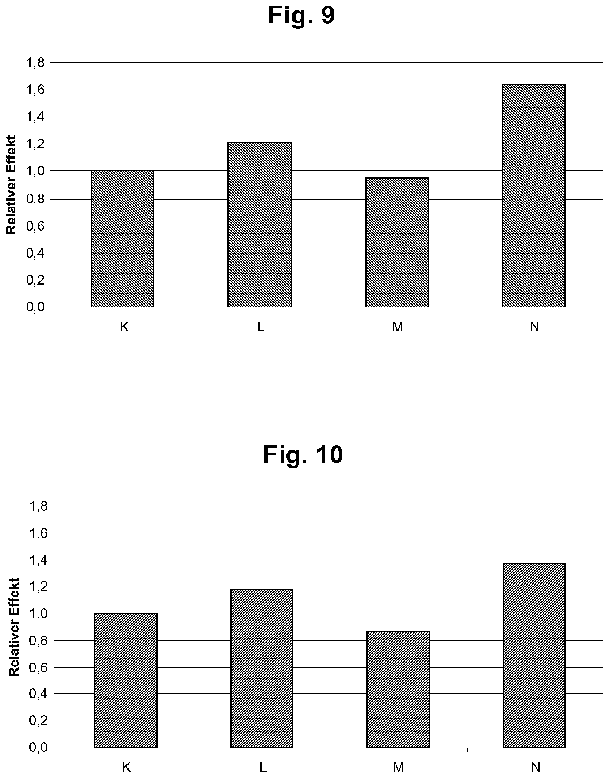

Die Expression von Kollagen-RNA (Figur 9) und Aggrecan-RNA (Figur 10) zeigt ein ähnliches Bild. Die Steigerung der Expression gegenüber der Kontrolle ist bei dem Ansatz N etwa drei Mal so hoch (Kollagen-RNA) bzw. etwa doppelt so hoch (Aggrecan-RNA) wie bei dem Ansatz L.

Um den Einfluss der Menge des eingesetzten Hagebuttenpulvers zu bestimmen, wurde eine Versuchsreihe mit folgenden Ansätzen von Zellkulturen durchgeführt:

| | |
|---|---|
| Ansatz O: | Kontrolle (ohne Kollagenhydrolysat oder Hagebuttenpulver) |
| Ansatz P: | 0,5 mg/ml Kollagenhydrolysat |
| Ansatz Q: | 0,5 mg/ml Kollagenhydrolysat und 0,005 mg/ml Hagebuttenpulver |
| Ansatz R: | 0,5 mg/ml Kollagenhydrolysat und 0,05 mg/ml Hagebuttenpulver |
| Ansatz S: | 0,5 mg/ml Kollagenhydrolysat und 0,5 mg/ml Hagebuttenpulver |

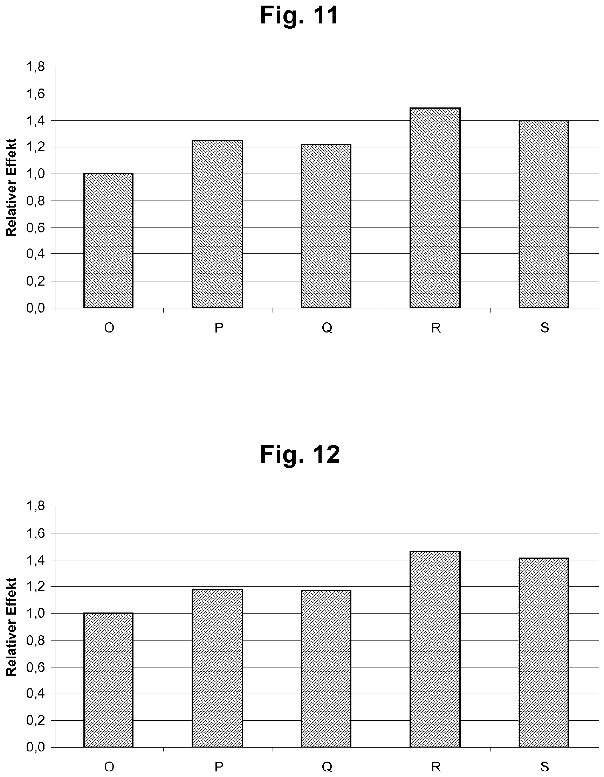

Die Figur 11 zeigt die Ergebnisse in Bezug auf die Biosynthese von Kollagen und die Figur 12 in Bezug auf die Biosynthese von Proteoglykanen. Bei dem Ansatz Q (Gewichtsverhältnis von Kollagenhydrolysat zu Hagebuttenpulver von 100:1) zeigt sich ein nur geringfügig höherer bzw. niedrigerer Effekt als bei dem Ansatz P (nur Kollagenhydrolysat), d.h. eine Steigerung der Biosynthese im Bereich von ca. 20%. Bei dem Ansatz R (Gewichtsverhältnis von 10:1) wird wiederum jeweils eine signifikante Steigerung der Biosynthese um ca. 50% beobachtet, entsprechend dem obigen Ansatz N. Eine weitere Erhöhung des Anteils an Hagebuttenpulver auf ein Gewichtsverhältnis von 1:1 (Ansatz S) führt sowohl bei der Kollagen-Biosynthese als auch bei der Proteoglykan-Biosynthese zu einer etwas geringeren Steigerung als bei dem Ansatz R.

Die vorstehend beschriebenen Ergebnisse belegen, dass durch die Zugabe von Hagebuttenextrakt oder Hagebuttenpulver die stimulierende Wirkung von Kollagenhydrolysat auf die Biosynthese der Hauptbestandteile der extrazellulären Matrix des Knorpelgewebes signifikant verstärkt werden kann. Eine Zusammensetzung, die Kollagenhydrolysat und Hagebuttenpulver und/oder -extrakt im Gewichtsverhältnis von ca. 2:1 bis ca. 100:1 enthält, kann daher als wirkungsvolles Arzneimittel oder Nahrungsergänzungsmittel zur Behandlung oder Vorbeugung von degenerativen Gelenkerkrankungen eingesetzt werden.
## Patentansprüche

1. Zusammensetzung, umfassend Kollagenhydrolysat und Hagebuttenpulver und/oder -extrakt, wobei das Gewichtsverhältnis von Kollagenhydrolysat zu Hagebuttenpulver und/oder -extrakt, jeweils bezogen auf die Trockenmasse, im Bereich von ca. 10:1 bis ca. 20:1 liegt.

2. Zusammensetzung nach Anspruch 1, wobei das Kollagenhydrolysat durch enzymatische Hydrolyse von Kollagen des Typs I und/oder des Typs II gewonnen ist.

3. Zusammensetzung nach einem der vorangehenden Ansprüche, wobei das Kollagenhydrolysat ein mittleres Molekulargewicht von ca. 0,3 kDa bis ca. 30 kDa aufweist, insbesondere von ca. 2 kDa bis ca. 10 kDa.

4. Zusammensetzung nach einem der vorangehenden Ansprüche, umfassend ein Hagebuttenpulver aus *Fructus cynosbati* und/oder *Semen cynosbati.*

5. Zusammensetzung nach Anspruch 4, wobei das Hagebuttenpulver durch Trocknen und Mahlen von *Fructus cynosbati* gewonnen ist.

6. Zusammensetzung nach einem der Ansprüche 1 bis 3, umfassend einen Hagebuttenextrakt aus *Fructus cynosbati* und/oder *Semen cynosbati.*

7. Zusammensetzung nach Anspruch 6, wobei der Hagebuttenextrakt durch Extraktion von *Fructus cynosbati* mit einem wässrigen Extraktionsmittel, welches bis zu 50 Vol.% Ethanol enthält, gewonnen ist.

8. Zusammensetzung nach Anspruch 7, wobei der Hagebuttenextrakt durch anschließende Enzymbehandlung und/oder Membranfiltration gewonnen ist.

9. Zusammensetzung nach Anspruch 8, wobei die Enzymbehandlung eine Behandlung des Hagebuttenextrakts mit einem oder mehreren Enzymen umfasst, die ausgewählt sind aus Glykosidasen, Cellulasen und Pektinasen.

10. Zusammensetzung nach Anspruch 8 oder 9, wobei die Membranfiltration eine Filtration des Hagebuttenextrakts bei einer Ausschlussgröße von ca. 1 kDa bis ca. 500 kDa, insbesondere von ca. 10 kDa bis ca. 300 kDa, umfasst.

11. Arzneimittel oder Nahrungsergänzungsmittel, umfassend eine Zusammensetzung nach einem der vorangehenden Ansprüche, insbesondere zur Behandlung oder Vorbeugung von degenerativen Gelenkerkrankungen.

12. Arzneimittel oder Nahrungsergänzungsmittel nach Anspruch 11, wobei die Gelenkerkrankung ausgewählt ist aus Arthrose, rheumatoider Arthritis, Erkrankungen des rheumatischen Formenkreises, Spondylitis und Fibromyalgie.

13. Arzneimittel oder Nahrungsergänzungsmittels nach Anspruch 11 oder 12 zur Stimulierung der Bildung von Knorpelgewebe.

14. Arzneimittel oder Nahrungsergänzungsmittel nach einem der Ansprüche 11 bis 13 in Form einer Lösung, eines Sirups, eines Pulvers oder eines Granulats oder in Form von Tabletten, Filmtabletten, Kapseln, Pastillen oder Dragees.

15. Arzneimittel oder Nahrungsergänzungsmittel nach Anspruch 14, wobei eine Dosis des Arzneimittels oder des Nahrungsergänzungsmittels ca. 0,1 g bis ca. 20 g Kollagenhydrolysat umfasst, insbesondere ca. 1 g bis ca. 10 g.

## Claims

1. Composition comprising collagen hydrolysate and rosehip powder and/or extract, wherein the weight ratio of collagen hydrolysate to rosehip powder and/or extract, in each case in relation to dry mass, is in the range of approximately 10:1 to approximately 20:1.

2. Composition according to claim 1, wherein the collagen hydrolysate is obtained by enzymatic hydrolysis of type I and/or type II collagen.

3. Composition according to one of the preceding claims, wherein the collagen hydrolysate has an average molecular weight of approximately 0.3 kDa to approximately 30 kDa, in particular approximately 2 kDa to approximately 10 kDa.

4. Composition according to one of the preceding claims, comprising a rosehip powder composed of *Fructus cynosbati* and/or *Semen cynosbati.*

5. Composition according to claim 4, wherein the rosehip powder is obtained by drying and milling *Fructus cynosbati.*

6. Composition according to one of claims 1 to 3, comprising a rosehip extract composed of *Fructus cynosbati* and/or *Semen cynosbati.*

7. Composition according to claim 6, wherein the rosehip extract is obtained by the extraction of *Fructus cynosbati* with an aqueous extracting agent containing up to 50% by vol. of ethanol.

8. Composition according to claim 7, wherein the rosehip extract is obtained by subsequent enzyme treatment and/or membrane filtration.

9. Composition according to claim 8, wherein the enzyme treatment comprises a treatment of the rosehip extract with one or more enzymes, which are selected from glycosidases, cellulases and pectinases.

10. Composition according to claim 8 or 9, wherein the membrane filtration comprises a filtration of the rosehip extract with an exclusion size of approximately 1 kDa to approximately 500 kDa, in particular approximately 10 kDa to approximately 300 kDa.

11. Medication or food supplement comprising a composition according to one of the preceding claims, in particular for the treatment or prevention of degenerative joint diseases.

12. Medication or food supplement according to claim 11, wherein the joint disease is selected from arthrosis, rheumatoid arthritis, rheumatic-type diseases, spondylitis and fibromyalgia.

13. Medication or food supplement according to claim 11 or 12 for stimulating the formation of cartilage tissue.

14. Medication or food supplement according to one of claims 11 to 13 in the form of a solution, a syrup, a powder or a granulate, or in the form of tablets, film tablets, capsules, lozenges or sugar-coated pills.

15. Medication or food supplement according to claim 14, wherein a dose of the medication or the food supplement comprises approximately 0.1 g to approximately 20 g of collagen hydrolysate, in particular approximately 1 g to approximately 10 g.

## Revendications

1. Composition, comprenant de l'hydrolysat de collagène et de la poudre et/ou de l'extrait de cynorrhodon, dans laquelle le rapport en poids d'hydrolysat de collagène à la poudre et/ou à l'extrait de cynorrhodon, respectivement par rapport à la masse sèche, se situé dans la plage d'environ 10:1 à environ 20:1.

2. Composition selon la revendication 1, dans laquelle l'hydrolysat de collagène est obtenu par hydrolyse enzymatique de collagène de type I et/ou de type II.

3. Composition selon l'une des revendications précédentes, dans laquelle l'hydrolysat de collagène présente un poids moléculaire moyen d'environ 0,3 kDa à environ 30 kDa, en particulier d'environ 2 kDa à environ 10 kDa.

4. Composition selon l'une des revendications précédentes, comprenant une poudre de cynorrhodon de *Fructus cynosbati* et/ou de *Semen cynosbati.*

5. Composition selon la revendication 4, dans laquelle la poudre de cynorrhodon est obtenue par séchage et broyage de *Fructus cynosbati.*

6. Composition selon l'une des revendications 1 à 3, comprenant un extrait de cynorrhodon de *Fructus cynosbati* et/ou de *Semen cynosbati.*

7. Composition selon la revendication 6, dans laquelle l'extrait de cynorrhodon est obtenu par extraction de *Fructus cynosbati* avec un agent d'extraction aqueux qui contient jusqu'à 50 % en volume d'éthanol.

8. Composition selon la revendication 7, dans laquelle l'extrait de cynorrhodon est obtenu par traitement enzymatique consécutif et/ou par filtration sur membrane.

9. Composition selon la revendication 8, dans laquelle le traitement enzymatique comprend un traitement de l'extrait de cynorrhodon avec une ou plusieurs enzymes qui sont choisies parmi les glycosidases, les cellulases et les pectinases.

10. Composition selon la revendication 8 ou 9, dans laquelle la filtration sur membrane comprend une filtration de l'extrait de cynorrhodon à une taille d'exclusion d'environ 1 kDa à environ 500 kDa, en particulier d'environ 10 kDa à environ 300 kDa.

11. Médicament ou complément alimentaire, comprenant une composition selon l'une des revendications précédentes, en particulier pour le traitement ou la prévention de maladies articulaires dégénératives.

12. Médicament ou complément alimentaire selon la revendication 11, dans lequel la maladie articulaire est choisie parmi l'arthrose, la polyarthrite rhumatoïde, des maladies de type rhumatismal, la spondylite et la fibromyalgie.

13. Médicament ou complément alimentaire selon la revendication 11 ou 12, pour stimuler la formation de cartilage.

14. Médicament ou complément alimentaire selon l'une des revendications 11 à 13 sous la forme d'une solution, d'un sirop, d'une poudre ou d'un granulé ou sous la forme de comprimés, de comprimés pelliculés, de capsules, de pastilles ou de dragées.

15. Médicament ou complément alimentaire selon la revendication 14, dans lequel une dose du médicament ou du complément alimentaire est d'environ 0,1 g à environ 20 g d'hydrolysat de collagène, en particulier d'environ 1 g à environ 10 g.


Sequenzprotokoll
<110> GELITA AG
<120> Zusammensetzung zur Behandlung von degenerativen Gelenkerkrankungen
<130> A 6 467 g
<160> 6
<210> 1
   <211> 20
   <212> DNA
   <213> porcin
<400> CCACTGCTCT CTGCCATACA
<210> 2
   <211> 20
   <212> DNA
   <213> porcin
<400> GTCCAGGTAG GCAATGCTGT
<210> 3
   <211> 20
   <212> DNA
   <213> porcin
<400> GGAGAAGAGA TGCCAACAGC
<210> 4
   <211> 20
   <212> DNA
   <213> porcin
<400> ATGCTGCTCA GGTGTGACTG
<210> 5
   <211> 20
   <212> DNA
   <213> porcin
<400> GGGCATGAAC CATGAGAAGT
<210> 6
   <211> 20
   <212> DNA
   <213> porcin
<400> AAGCAGGGAT GATGTTCTGG